# EUROPÄISCHE PATENTSCHRIFT

(11) **EP 1 454 137 B1**
(45) Veröffentlichungstag und Bekanntmachung des Hinweises auf die Patenterteilung: **13.02.2008**
(21) Anmeldenummer: 02804582.1
(22) Anmeldetag: 09.12.2002
(51) Int. Cl.: G01N 33/569

(54) **VERWENDUNG EINES MARKIERTEN LIGANDEN MIT SPEZIFITÄT FÜR DAS HUMANE CD4-MOLEKÜL**
USE OF A LABELED LIGAND HAVING HUMAN CD4 SPECIFICITY FOR PRODUCING A DIAGNOSTIC USED IN THE ANALYSIS OF MIGRATION AND/OR DISTRIBUTION PATTERNS OF CELL POPULATIONS
UTILISATION D'UN LIGAND MARQUE PRESENTANT UNE SPECIFICITE POUR LA MOLECULE CD4 HUMAINE AFIN D'ETABLIR UN DIAGNOSTIC EN VUE D'ANALYSER DES MODELES DE MIGRATION ET/OU DE DISTRIBUTION DE POPULATIONS CELLULAIRES

(30) Priorität: 11.12.2001 DE 10160653; 16.07.2002 DE 10232189
(43) Veröffentlichungstag der Anmeldung: 08.09.2004
(73) Patentinhaber: BIOTECTID GmbH, 04103 Leipzig (DE)
(72) Erfinder: EMMRICH, Frank, 04299 Leipzig (DE); KINNE, Raimund, W., 07743 Jena (DE); LAUB, Rüdiger, 04277 Leipzig (DE); PIGLA, Ullrich, D-04275 Leipzig (DE)
(74) Vertreter: Dey, Michael
(86) Internationale Anmeldenummer: PCT/EP2002/013939
(87) Internationale Veröffentlichungsnummer: WO 2003/050499

(56) Entgegenhaltungen:
- EP-A- 1 118 858
- US-A- 6 146 614
- BECKER W ET AL: "IMAGING RHEUMATOID ARTHRITIS SPECIFICALLY WITH TECHNETIUM-99M CD4-SPECIFIC T-HELPER LYMPHOCYTES ANTIBODIES" EUROPEAN JOURNAL OF NUCLEAR MEDICINE, Bd. 17, Nr. 3-4, 1990, Seiten 156-159, XP008020066 ISSN: 0340-6997
- LAUB R ET AL: "A multiple transgenic mouse model with a partially humanized activation pathway for helper T cell responses" JOURNAL OF IMMUNOLOGICAL METHODS, ELSEVIER SCIENCE PUBLISHERS B.V.,AMSTERDAM, NL, Bd. 246, Nr. 1-2, 1. Dezember 2000 (2000-12-01), Seiten 37-50, XP004220739 ISSN: 0022-1759 in der Anmeldung erwähnt
- KRETZSCHMAR ET AL: "In vivo binding of 99m Tc-Labelled monoclonal antibody HYNIC-Fab in mice - A quantitative whole-body autoradiographic study" FORSCHUNGSBERICHT DES FZ ROSSENDORF, 2003,

## Beschreibung

Die Erfindung betrifft die Verwendung eines markierten. Liganden mit Spezifität für das humane CD4-Molekül ausgewählt aus der Gruppe bestehend aus Antikörperfragmenten ausgewählt aus F(ab')2, Fab' und Fab des Antikörpers Max.16H5 und rekombinanten Antikörperfragmenten ausgewählt aus F(ab')2, Fab' und Fab des Antikörpers Max.16H5 zur Herstellung eines in vivo Diagnostikums zur Analyse von Wanderungs- und/oder Verteilungsmustern bestimmter Zellpopulationen, die CD4-tragende Zellen umfassen, in menschlichen Individuen.

Das monomere Glykoprotein CD4 ist das charakteristische Zelloberflächenmolekül von Helfer T-Lymphozyten. Es hat ein Molekulargewicht von 55 kDa und besteht aus einer extrazellulären Region, einem hydrophoben transmembranösen sowie einem zytoplasmatischen Teil. Humanes CD4 wird nicht nur auf menschlichen Helfer T-Lymphozyten exprimiert, sondern in geringerer Dichte auch auf T-Lymphozyten-Vorläufern im Knochenmark und im Thymus. Beim Menschen tragen auch Monozyten, Makrophagen, dendritische Zellen und eosinophile Granulozyten das CD4-Molekül [Marsh & Pelchen-Matthews, 1996]. Das humane CD4-Gen ist auf dem kurzen Arm des Chromosoms 12 lokalisiert [Isobe et al., 1986].

Das CD4-Molekül wird aufgrund seiner vier lmmunglobulin-ähnlichen extrazellulären Domänen (D1 ― D4) der lmmunglobulinsuperfamilie zugeordnet [Maddon et al., 1985]. Die aminoterminale Domäne 1 (D1) besitzt eine ähnliche Struktur wie eine variable Immunglobulindomäne. Gemeinsam mit Domäne 2 (D2) bildet sie eine starre Struktur mit 60 Angstrom Länge. Die Domänen D1 und D2 sind über ein flexibles Gelenk mit den Domänen D3 und D4 verbunden.

Wichtigster natürlicher CD4-Ligand sind Klasse-II-Moleküle des Haupthistokompatibilitätskomplexes (major histocompatibility complex, MHC) auf sogenannten Antigen-präsentierenden Zellen (APZ). Dabei handelt es sich um spezialisierte Zellen des Immunsystems, die antigene Strukturen aufbereiten und diese für Immunantworten erkennbar machen. Die extrazellulären Domänen D1 und D2 des CD4-Moleküls assoziieren hauptsächlich mit nicht-polymorphen Regionen der 2-Kette des MHC-Klasse-II-Moleküls [Clayton et al., 1989; Cammarota et al., 1992]. Möglicherweise ist auch die a2-Kette des MHC-Klasse-II-Moleküls (MHC-II) für die Interaktion mit CD4 bedeutsam [Vignali et al., 1996].

Über ihre D1-Domänen können CD4-Moleküle miteinander assoziieren, wodurch funktionelle Oligomere entstehen, die den T-Zellrezeptor (TZR)/MHC-II-Peptid-Komplex stabilisieren und die Effizienz der Signaltransduktion erhöhen [Li et al., 1998]. Über die proximalen Domänen D3 und D4 kann CD4 auch mit dem TZR/CD3-Komplex assoziieren [Vignali et al., 1996]. Das CD4-Molekül verstärkt die Antigen-spezifische Stimulation von T-Zellen während der Interaktion des TZR mit dem Antigenpeptid-präsentierenden MHC-II. Das CD4-Corezeptor-System ermöglicht somit nicht nur eine Erhöhung der Antigen-Sensitivität, sondern auch die Aufrechterhaltung der hohen Antigen-Spezifität [König et al., 1996].

CD4 ist aktiv an der Signaltransduktion in die T-Lymphozyten beteiligt. Seine zytoplasmatische Domäne ist nicht-kovalent mit einer T-zellspezifischen Protein-Tyrosinkinase (p561ck) der sogenannten src-Familie assoziiert [Veilette et al., 1988]. Der proximale Teil der zytoplasmatischen CD4-Domäne interagiert über Cysteinreste mit der N-terminalen Region der p561ck [Turner et al., 1990].

Weitere natürliche CD4-Liganden für humanes CD4 sind das gpl2O des humanen Immundefizientvirus (HIV), das chemotaktische Zytokin IL-16 sowie Immunglobuline. Die Bindungsstellen für gpl20 und MHC-II sind nicht identisch, liegen jedoch nah beieinander und überlappen teilweise. HIV gpl20 bindet hauptsächlich an die D1 Domäne des CD4-Moleküls [Clayton et al., 1989]. Die Infektion von CD4+-Zellen mit HIV führt zum Verlust der CD4-Expression auf der Zelloberfläche [Hoxie et al., 1986a]. Dadurch wird die Fähigkeit infizierter Zellen, mit MHC-II der APZ zu interagieren, eingeschränkt, und das Fortschreiten der Erkrankung begünstigt.

Chronische und chronisch-rezidivierende Entzündungen treten im Zusammenhang mit vielen Erkrankungen auf bei denen das Immunsystem von Bedeutung ist. Von chronisch rezividierenden Entzündungen können unterschiedliche Organe bzw. Gewebe betroffen sein. Im Gegensatz zu akuten Entzündungen akkumulieren in den Entzündungsherden grundsätzlich qualitativ andere Zelltypen. Diese können anhand spezifischer Oberflächenmoleküle identifiziert werden. Eines dieser Moleküle ist das humane CD4-Molekül, das unter anderem auf Helfer-T-Lymphozyten, Monozyten, Makrophagen, dendritischen Zellen und eosinophilen Granulozyten in unterschiedlicher Stärke exprimiert wird. Diese Zellen, insbesondere die T-Lymphozyten, stellen eine wesentliche Population chronischer Entzündungszellen dar.

Bestimmte monoklonale Antikörper können das humane CD4-Molekül hochspezifisch erkennen, an dessen Proteinstruktur binden und somit human-CD4 exprimierende Zellen in der Entzündung hochspezifisch binden.

Unter physiologischen Bedingungen liegen diese Entzündungszellen in einem inaktiven Differenzierungszustand vor und folgen charakteristischen Wanderungs- und/oder Verteilungsmustern in primären und sekundären lymphatischen Organen sowie dem zirkulierenden Blut.

Außerdem können sich aus human-CD4-tragenden Zellen Tumorzellen entwickeln, die Anlaß für die Entstehung von malignen oder benignen Tumorerkrankungen geben. Bei bestimmten Tumorerkrankungen, z.B. bestimmten Lymphomen, kann dabei die Expression des humanen CD4-Moleküls erhalten bleiben.

Die qualitative und quantitative Erfassung CD4-exprimierender Zellen ist daher eine etablierte immundiagnostische in vitro-Methode. Während die flusszytometrische Analyse eine mengenmässige Erfassung im Peripherblut ermöglicht, können mit immunhistologischen Verfahren ex vivo, d.h., nach Biopsie verdächtiger Gewebe, ihre lokalen Verteilungsmuster analysiert werden.

Beide Methoden sind jedoch nicht in der Lage, eine objektive Aussage zu Wanderungs- und/oder Verteilungsmustern der T-Helferzellen im lebenden Organismus bzw. zur möglichen Akkumulation und deren Ausmaß in erkrankten Geweben/Organen zu liefern. Das hat folgende Ursachen:
(1) Peripherblut enthält mit maximal 5 % einen nur begrenzt repräsentativen Anteil aller human-CD4-exprimierenden Zellen des Körpers;
(2) in jedem Individuum bestehen beträchtliche Variabilitäten in den Zahlen peripherer human-CD4-exprimierender Zellen von Tag zu Tag;
(3) die peripheren human-CD4-exprimierenden Zellanzahlen ermöglichen keineswegs die Identifizierung und die Lokalisierung räumlich begrenzter Akkumulationen in chronischen Entzündungsherden oder die Lokalisierung entarteter human-CD4-exprimierender Zellen in Tumoren;
(4) die Wahrscheinlichkeit, dass eine Biopsie den für die Diagnostik entscheidenden Bereich eines Entzündungsherdes oder Tumors oder einer Metastase trifft, ist nur gering;
(5) aus dem Biopsiematerial kann nicht auf die tatsächliche räumliche Ausdehnung des Entzündungsgebietes oder des Tumors oder der Metastase geschlossen werden; und
(6) eine Verlaufs- bzw. Therapiekontrolle ist nur durch wiederholte Biopsien möglich und bringt eine erhebliche Belastung für den Betroffenen mit sich.

Becker et al (1990) beschreibt die Verwendung des markierten Komplettantikörpers Max.16H5 zur Analyse von Wanderungs - und/oder Verteilungsmustern von Zellpopulationen, die human-CD4-tragende Zellen umfassen, in menschlichen Individuen.

US 6,146,614 beschreibt ein Verfahren zur Bestimmung der Lymphozytenverteilung und -wanderung in Säugern unter Verwendung von bildgebenden Verfahren und die Verwendung dieser Bestimmungen zur Diagnose des Verlaufs verschiedener Krankheiten sowie zum Überwachen der Antwort auf verschiedene Therapien für solche Erkrankungen und zum Identifizieren von Arzneimitteln zur Behandlung dieser Erkrankungen. Die Beispiele der US 6,146,614 betreffen die Verwendung von monoklonalen Antikörpern gegen Maus-CD4-Antigen. Ein Beispiel betrifft die Verwendung eines solchen Antikörpers zur Markierung von menschlichen Zellen. Dieses Beispiel steht jedoch in Widerspruch zu der allgemein bekannten Tatsache, dass es keine Kreuzreaktivität zwischen humanem CD4 und murinem CD4 gibt. Beispiel 7 der vorliegenden Anmeldung zeigt daher einen entsprechenden Vergleichsversuch, aus dem klar hervorgeht, dass der in US 6,146,614 beschriebene Antikörper gegen murines CD4 keine Kreuzreaktionen zu humanem CD4 aufweist. Das in US 6,146,614 beschriebene Verfahren eignet sich daher nicht zur Bestimmung des Verteilungs- und Wanderungsmusters von CD4-tragenden Zellen in menschlichen Individuen.

Aufgrund der oben beschriebenen Beschränkungen der herkömmlichen diagnostischen Methoden besteht eine dringende Notwendigkeit für ein Verfahren zur nicht-invasiven Bestimmung der Wanderungs- und/oder Verteilungsmuster und der Akkumulation von Entzündungszellen und Tumorzellen, die CD4-Oberflächenmoleküle tragen, sowie ihrer Redistribution bzw. Auflösung unter Therapiebedingungen (Therapiekontrolle).

Der Erfindung liegt daher die Aufgabe zugrunde die vorstehend beschriebenen Nachteile des Standes der Technik zu überwinden. Insbesondere ist es Aufgabe der Erfindung, die Wanderungs- und/oder Verteilungsmuster von human-CD4-exprimierenden Zellen in menschlichen Individuen unter Vermeidung invasiver diagnostischer Maßnahmen bestimmen zu können.

Einer weitere Aufgabe der Erfindung ist es, sicher, effektiv und einfach die Wanderungs- und/oder Verteilungsmuster von human-CD4-exprimierenden Zellen in Patienten mit (Verdachts-)Diagnose auf chronisch-inflammatorische Erkrankungen, Infektionserkrankungenoder Tumorerkrankungen oder den Verlauf einer immunsupressiven Therapie nach einer Transplantation in menschlichen Individuen zu bestimmen.

Eine weitere Aufgabe der Erfindung ist es, die Unterscheidung chronischer Entzündungsherde, wie bei Autoimmunerkrankungen, und akuter Entzündungen, wie infektionsbedingter Entzündungen, basierend auf der Erkenntnis zu ermöglichen, dass in chronischen Entzündungsinfiltraten mehr Zellen mit besagtem Oberflächenantigen vorliegen als in akuten Entzündungsinfiltraten. So sind in der chronisch entzündeten Synovialmembran bei rheumatoider Arthritis vermehrt CD4-exprimierende Zellen nachweisbar. Das CD4/CD8-Verhältnis ist hier 2- bis 7-fach gegenüber dem des Peripherbluts erhöht [Immunology of rheumatic diseases. Hrsg. S. Gupta & N. Talal. Plenum Medical Book Company, New York und London, 1985]. Darüber hinaus hat die antigenspezifische Aktivierung von Helfer-T-Lymphozyten eine deutliche Steigerung der CD4-Oberflächenexpression zur Folge [Ridgeway et al., 1998, J. Immunology 161:714-720].

Eine weitere Aufgabe der Erfindung ist es, die Lokalisierung und die Bestimmung der räumlichen Ausdehnung und der Intensität von Entzündungsherden bei Autoimmunerkrankungen zu ermöglichen sowie den Verlauf von Autoimmunerkrankungen oder den Einfluss von therapeutischen Maßnahmen bei Autoimmunerkrankungen zu beurteilen.

Eine weitere Aufgabe der Erfindung ist es, sicher, effektiv und einfach Tumore, die humanes CD4 exprimieren, beziehungsweise deren Metastasen in menschlichen Individuen mit (Verdachts-)Diagnose auf maligne und benigne Erkrankungen des hämatopoetischen Systems zu lokalisieren, die räumliche Ausdehnung von human-CD4-tragenden Tumoren beziehungsweise deren Metastasen zu bestimmen und den Verlauf von Tumorerkrankungen (Verlaufskontrolle) und therapeutischen Maßnahmen bei Tumorerkrankungen (Therapiekontrolle) zu beurteilen.

Erfindungsgemäß werden diese Aufgaben gelöst durch die Verwendung eines markierten Liganden mit Spezifität für das humane CD4-Molekül ausgewählt aus der Gruppe bestehend aus Antikörperfragmenten ausgewählt aus F(ab')2, Fab' und Fab des Antikörpers Max.16H5 und rekombinanten Antikörperfragmenten ausgewählt aus F(ab')2, Fab' und Fab des Antikörpers Max.16H5 zur Herstellung eines Diagnostikums zur Analyse von Wanderungs- und/oder Verteilungsmustern von bestimmten Zellpopulationen, die human-CD4-tragende Zellen umfassen, in menschlichen Individuen.

Die räumliche Lage bzw. Anordnung human-CD4-tragender Zellen in Zellpopulationen im menschlichen Organismus wird im Sinne der vorliegenden Erfindung als Verteilungsmuster bezeichnet. Die Lage oder Anordnung human-CD4-exprimierender Zellen betrifft deren Verteilung in den eigenen Geweben oder in eingebrachten fremden Geweben und synthetischen Materialien bzw. deren Anordnung an den Grenzflächen besagter fremder Gewebe und synthetischer Materialien. Die Verteilung human-CD4-tragender Partikel entsteht nach deren Verabreichung in den menschlichen Organismus. Die Verteilungsmuster können sowohl für den gesamten Organismus als auch für Teile des Organismus bestimmt werden.

Für die human-CD4-tragenden Partikel kann das humane CD4-Molekül in rekombinanten Produktionssystemen hergestellt werden (recCD4). Mit recCD4 können dann geeignete Träger unterschiedlicher Größe beschichtet werden. Trägermaterialien können Kunststoffpartikel sein, aber auch Gebilde, die von Membranen umschlossen sind und im Inneren therapeutisch aktive Substanzen enthalten. So kann das humane CD4-Molekül als Erkennungsstruktur (guide) an Trägern oder Partikeln oder Vesikeln, wie z.B. Liposomen, angebracht werden, um diese z.B. gefüllt mit pharmakologischen Substanzen an Zielzellen heranzubringen, die wiederum Moleküle tragen, die mit CD4 interagieren. Bei diesen Molekülen handelt es sich um Mitglieder der MHC-Klasse-II-Antigene (beim Menschen HLA-Klasse-II-Antigene), die auf verschiedenen Körperzellen exprimiert sind, wie z.B. Monozyten, Makrophagen, dendritische Zellen, B-Zellen, und auf einer Reihe weiterer Zellen unter Einwirkung von Milieufaktoren, wie z.B. bestimmte Zytokine, aufreguliert werden können. Da die Heraufregulierung der MHC-Klasse-II-Moleküle in Zusammenhang mit Pathogeneseprozessen gebracht wird, ergibt sich die Möglichkeit, human-CD4-geleitet pharmakologisch wirksame Substanzen in bestimmte Gewebezonen zu dirigieren. CD4-tragende Partikel können beispielsweise für die Auffindung komplementärer Strukturen, z.B. für das Oberflächenprotein gp 120 von HIV, eingesetzt werden und eine lokale Anreicherung von antiviralen Wirkstoffen vermitteln. Es ist jedoch auch denkbar, Anti-CD4-Antikörper oder Antikörperfragmente als molekulare "guides" an Partikel, Moleküle oder Vesikel zu binden, die dann in CD4-tragende Gewebebezirke dirigiert werden.

Unter Wanderungsmuster der human-CD4-tragenden Zellen bzw. Partikel im Sinne der vorliegenden Erfindung wird die Bewegung der human-CD4-tragenden Zellen oder Partikel im menschlichen Organismus, einschließlich seiner Gewebe und des peripheren Blutes verstanden. Die Wanderung der human-CD4-tragenden Zellen oder Partikel kann für den gesamten Organismus oder bestimmte Teile oder Organe desselben bestimmt werden.

Human-CD4-exprimierende Zellen im Sinne der vorliegenden Erfindung können Monozyten, Makrophagen, dendritische und Langerhans-Zellen, eosinophile Granulozyten und Helfer-T-Lymphozyten umfassen. In bestimmten Fällen sind diese Zellen in der Zusammensetzung von entzündlichen Infiltraten bei Autoimmunerkrankungen, Infektionserkrankungen, malignen Erkrankungen oder bei Transplantationen bzw. Implantationen enthalten. In weiteren Fällen sind diese Zellen in ihrer Regulation verändert (entartet), dass sie Tumoren bilden. Weitere Fälle betreffen Komplikationen nach Transplantation von Organen oder Gewebe oder nach Implantation synthetischer Ersatzstoffe. Gemeint sind Komplikationen die zum Verlust des Tranplantates oder Implantates führen können (immunologische Abstoßungsreaktionen).

Eine Substanz/Verbindung, die spezifisch mit CD4-tragenden Zellen interagieren kann, wird im Sinne der vorliegenden Offenbarung als Ligand bezeichnet. Ein Ligand kann z.B. mit bestimmten Molekülen, die auf der Oberfläche von Zellen exprimiert werden oder auf der Oberfläche von Partikeln befestigt sind, interagieren, in diesem Fall mit CD4-Molekülen. Der Ligand kann jede beliebige Substanz oder Verbindung mit der Eigenschaft sein, ausschließlich oder weit überwiegend mit dem CD4-Oberflächenmolekül zu interagieren und an dieses zu binden.

Das erfindungsgemäß verwendbare Diagnostikum umfasst einen markierten hochaffinen Liganden mit Spezifität für das humane CD4-Molekül ausgewählt aus der Gruppe bestehend aus Antikörperfragmenten ausgewählt aus F(ab')2, Fab' und Fab des Antikörpers Max.16H5 und rekombinanten Antikörperfragmenten ausgewählt aus F(ab')2, Fab' und Fab des Antikörpers Max.16H5. Besonders bevorzugt erkennt der markierte Ligand ein Epitop in der D1-Domäne des humanen CD4-Moleküls.

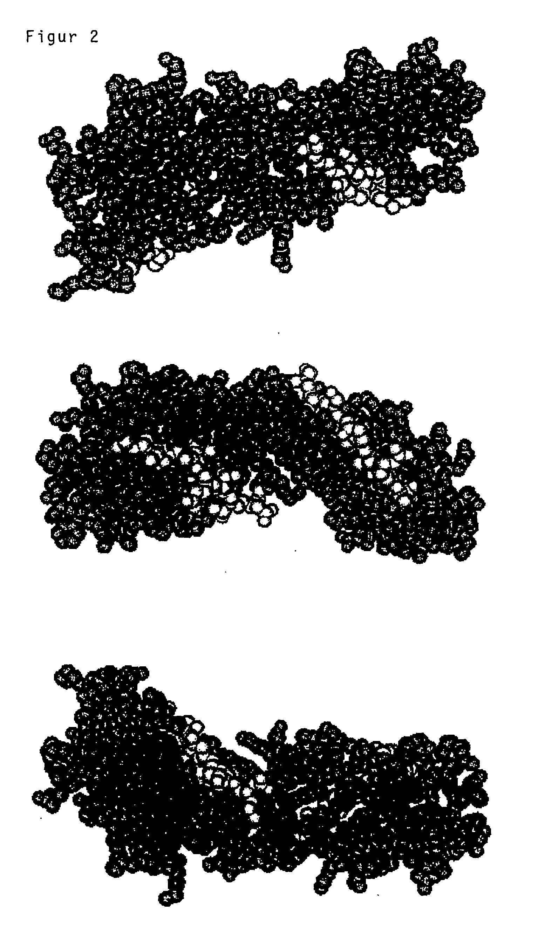

Weiterhin können die Antikörperfragmente oder rekombinanten Antikörperfragmente bevorzugt eine der in SEQ ID NO. 1 bis SEQ ID NO. 6 gezeigten Sequenzen erkennen und daran binden. Besonders bevorzugt erkennen die Antikörperfragmente oder rekombinanten Antikörperfragmente das Konformationsepitop (Figur 2) des humanen CD4 und binden daran. Die Antikörperfragmente oder rekombinanten Antikörperfragmente sein Anti-CD4-Antikörper.

Es gibt polyklonale oder monoklonale Antikörper insbesondere der monoklonale Antikörper Max.16H5. Gesamte Antikörper können allerdings immunogen sein und nach Verabreichung in einen menschlichen Organismus gegen sich selbst gerichtete Immunreaktionen induzieren. Kleinere Liganden sind weniger immunogen.

Die Erfindung verwendet kleinere Liganden, nämlich Antikörpenfragmente des monoklonalen Antikörpers Max.16H5 ausgewählt aus F(ab')₂, Fab' and Fab.

Ein Beispiel für einen kleineren Liganden ist OKT3 (Orthoklon). Orthoklon ist ein monoklonaler Anti-human-CD3-Antikörper, der durch Maushybridomzellen gebildet wird und von Ortho Pharmaceutical beispielsweise zur Behandlung von immunologischenAbstoßungsreaktionen nachTransplantationen verwendet wird. Andere monoklonale Antikörper oder Antikörperfragmente, z.B. gegen humanes CD4, können in an sich bekannter Weise unter Verwendung der Hybridomtechnik hergestellt werden.

Weitere Beispiele für Liganden sind Antikörper oder Antikörperfragmente, die mit rekombinanten Methoden in Mikroorganismen hergestellt werden, wie Einzelketten-Antikörper, beispielsweise single chain Fv (scFv), antikörperähnliche Strukturen, beispielsweise Minibodies, und weitere Fragmente, die spezifisch an ein Oberflächenmolekül, wie humanes CD4 (human-CD4) binden können, beispielsweise rekombinante Antikörper.

Weiterhin gibt es auch als Ligand Proteine, Glykoproteine, Peptide, Peptidomimetika, aliphatische und zyklische Kohlenwasserstoffverbindungen und Aptamere, die mit hoher Spezifität an ein Oberflächenmolekül, wie humanes CD4, binden. Peptidomimetikum bezeichnet eine Verbindung, die kein Peptid ist, diesem aber in Bezug auf wichtige Eigenschaften, wie Größe und Ladungsverteilung, ähnelt. Ein Aptamer ist eine DNA- oder RNA-Struktur mit hoher Affinität zu besagten Oberflächenmolekülen, z.B. zu humanem CD4.

Die Markierung des Liganden kann durch jedes Markierungsmolekül, das die zeitliche und räumliche Lokalisierung des markierten Liganden mit Verfahren der medizinischen Bildgebung ermöglicht, erfolgen.

Der markierte Ligand ist vorzugsweise ausgewählt aus der Gruppe, bestehend aus Gamma-Emittern, Positronen-Emittern, magnetischem Material, Dichtekontrastmaterial und Mischungen davon.

Der markierte Ligand kann ein Gamma-Emitter sein, der ein oder mehrere Radioisotope umfasst, ausgewählt aus der Gruppe, bestehend aus Indium-111, Technetium-99m, Technetium-99, Jod-132, anderen Radioisotopen für medizinisch bildgebende Verfahren und deren Mischungen. Das Radioisotop ist besonders bevorzugt Technetium-99m oder Indium-111.

In einer weiteren bevorzugten Ausführungsform ist der markierte Ligand ein Positronen-Emitter, der ein oder mehrere Isotope umfasst, ausgewählt aus der Gruppe, bestehend aus Fluor-18, Kohlenstoff-11, Jod-124, anderen Isotopen für medizinisch-bildgebende Verfahren und deren Mischungen.

Der markierte Ligand kann in einer weiteren bevorzugten Ausführungsform ein magnetisches Material umfassen, ausgewählt aus der Gruppe, bestehend aus paramagnetischen Substanzen, wie Gadolinium, superparamagnetischen Substanzen, hydratisierten Eisenoxidpartikeln, anderen Materialien für medizinisch-bildgebende Verfahren und deren Mischungen.

Der markierte Ligand kann auch ein Dichtekontrastmaterial umfassen.

Die human-CD4 tragenden Zellen sind vorzugsweise ausgewählt aus der Gruppe, bestehend aus T- und B-Lymphozyten, Monozyten, Makrophagen, dendritischen und Langerhans-Zellen und eosinophilen Granulozyten, insbesondere können die Lymphozyten aus einer Gruppe oder Population ausgewählt werden, die nicht-lymphozytäre Zellen umfasst.

Die Analyse der Wanderungs- und/oder Verteilungsmuster von Zellpopulationen, die human-CD4-tragende Zellen umfassen, kann durch die spezifische Interaktion des Diagnostikums mit CD4-tragenden Zellen nach Verabreichung des markierten Liganden in menschliche Individuen erfolgen. Die spezifische Interaktion des in vivo-Diagnostikums resultiert in markierten Zellen. Diese Zellen sind maßgeblich am Entzündungsinfiltrat, speziell bei chronischen Entzündungen beteiligt.

Alternativ kann die Analyse der Wanderungs- und Verteilungsmuster von Zellpopulationen, die human-CD4-tragende Zellen umfassen, durch spezifische Interaktion mit Zellpopulationen, die von einem menschlichen Organismus abstammen und CD4-tragende Zellen enthalten, ausserhalb des menschlichen Organismus erfolgen. Auch in diesem Fall resultiert die spezifische Interaktion in markierten Zellen, die in einen Organismus eingebracht werden. Eine Markierung von Zellen außerhalb des Körpers zur Verwendung beim gleichen Patienten, ist in der Nuklearmedizin durchaus üblich. Bisher werden jedoch auf diese Weise die Zellen direkt mit verschiedenen Isotopen markiert. Dies hat entweder technische Gründe (hohe Konzentration für die Markierung erforderlich), dient dem Gesundheitsschutz des Patienten (nicht gebundene Radioaktivität wird vor der Rückgabe in den Patienten entfernt) oder/und ökonomische Gründe (die kostbaren Produkte werden in sehr kleinen Volumina effektiv für die Markierung verwendet).

Die Markierung außerhalb des menschlichen Organismus kann dabei wie folgt erfolgen: Dem Patienten wird Blut entnommen. Aus diesem Blut werden Zellen präpariert und außerhalb des Körpers mit isotopenmarkierten Liganden, z.B. Anti-CD4-Antikörpern, inkubiert. Danach wird nicht gebundener Ligand enfernt und die gereinigte Zellpräparation dem gleichen Patienten wieder injiziert. Dann wird mit den bekannten und beschriebenen Verfahren (Gammakamera oder Detektorsonde) die Verteilung der markierten Zellen im Körper verfolgt. Interessant ist die Verwendung dieses Verfahrens beispielsweise, wenn markierte Zellen während der Operation in Tumore oder in die Umgebung von Tumoren injiziert werden. Sie suchen dann die abfließenden Lymphgefäße und die nachgeschalteten Lymphknoten auf. Auf diese Weise gelingt es mit einer bleistiftähnlichen Detektorsonde noch während der Operation festzustellen, in welche Lymphknoten diese Zellen vorzugsweise geströmt sind. Diese besonders frequentierten Lymphknoten sind auch der wahrscheinliche Ort der Metastasierung, sodass der Chirurg diese sofort entfernen kann.

Durch die erfindungsgemäße Verwendung des Diagnostikums können mit Hilfe medizinischer Bildgebungsverfahren die Wanderungs- und/oder Verteilungsmuster von Zellen, die CD4-Oberflächenmoleküle tragen, im Organismus bestimmt werden. Die Möglichkeit der Darstellung von Wanderungs- und/oder Verteilungsmustern unter normalen und pathologischen Bedingungen erlaubt, Ausmaß und Progredienz von Erkrankungen, bei denen besagte Zellen von klinischer Bedeutung sind, zu bestimmen.

Insbesondere können durch Analyse von Wanderungs- und/oder Verteilungsmustern von human-CD4-tragenden Zellen mit Hilfe der erfindungsgemäßen Verwendung des Diagnostikums chronische Entzündungsherde im menschlichen Organismus lokalisiert und von akuten Entzündungen unterschieden werden. Die Analyse von Wanderungs- und/oder Verteilungsmustern von human-CD4-tragenden Zellen eignet sich außerdem zur Abklärung von Verdachtsdiagnosen, zum Monitoring des klinischen Verlaufs und zur Erfolgskontrolle anti-entzündlicher Therapien.

Ein weiteres Anwendungsgebiet für die Analyse von Wanderungs- und/oder Verteilungsmustern von human-CD4-tragenden Zellen ist das Auffinden von bösartigen Zellen (Tumorzellen), auf denen besagte Oberflächenmoleküle exprimiert sind und die mit dem Diagnostikum interagieren, so dass markierte Tumorzellen entstehen. Insbesondere kann die Analyse von Wanderungs- und/oder Verteilungsmustern von human-CD4-tragenden Zellen der Lokalisierung von Tumoren des hämatopoetischen Systems beziehungsweise deren Metastasen dienen, sofern auf diesen besagtes Oberflächenmolekül exprimiert wird.

Des Weiteren kann mit Hilfe des Diagnostikums eine sogenannte Radioimmunotherapie (RIT) durchgeführt werden. Für die RIT werden die diagnostischen Radioisotope gegen solche mit starker *α*- oder/und *β*-Strahlung ausgestauscht, wie z.B. Re-186, Re-188, J-131, Y-90, Sm-153 als *β-*Emitter und Bi-213 als *α*-Emitter. Dabei kommen auch gleichartig derivatisierte Antikörper, deren Fragmente bzw. Liganden zur Anwendung. Für DTPA-derivatisierte Moleküle sind das z.B. In-111 und Y-90. Auch J-123 und J-131 sowie Tc-99 und Re-188 repräsentieren weitere Isotopenpaare für Diagnostik und Therapie. Die von therapeutisch angewendeten Radioisotopen emittierte *α*- oder/und *β*-Strahlung vernichtet Zellen, die sich in unmittelbarer Umgebung des Radioimmunotherapeutikums befinden bzw. von diesem markiert wurden. Auf diese Weise können markierte Tumorzellen bzw. schädliche Entzündungszellen oder andere unerwünschte Zellen selektiv vernichtet werden. Vorteilhaft ist hier, dass nicht nur die Zelle, die das CD4-Molekül trägt, sondern auch unmittelbar umgebende Zellen geschädigt oder zerstört werden. Nicht beeinträchtigt wird weiter entfernt liegendes Gewebe.

Die erfindungsgemäßen Diagnostika können darüber hinaus eingesetzt werden zur Darstellung und Quantifizierung von Entzündungszellen bei Infektionserkrankungen, z.B. Viruskrankheiten. Dadurch sind Stadienbeurteilungen, Verlaufs- und Therapiekontrollen, z.B. von Lymphknotenschwellungen, bestimmter Tumorerkrankungen und ein Monitoring antiviraler Therapien möglich.

Insbesondere ist die erfindungsgemäße Verwendung zur Analyse von Wanderungs- und Verteilungsmustern bestimmter Zellpopulationen in menschlichen Individuen bei Krankheiten oder Verdachtsdiagnosen für Krankheiten folgender Formenkreise geeignet:

Autoimmunerkrankungen, Infektionskrankheiten, maligne und benigne Erkrankungen des hämatopoetischen Systems, Infektionskrankheiten oder Erkrankungen, für die eine pathogenetische Bedeutung entweder CD4-tragender oder CD8-tragender T-Lymphozyten angenommen wird.

Autoimmunerkrankungen können rheumatoide Arthritis, multiple Sklerose, systemischer Lupus Erythematosus, Psoriasis oder eine entzündliche Darmerkrankung, wie Morbus Crohn oder Colitis Ulcerosa, sein. Maligne oder benigne Erkrankung können Erkrankungen des hämatopoetischen Systems, wie Myelom, Lymphom, oder Leukämie oder solide Tumoren, wie Melanom, Nierenzellkarzinom, Ovarialkarzinom oder Lungenkarzinom, sein. Infektionserkrankungen können alle Erkrankungen sein, die durch Bakterien oder Viren hervorgerufen werden, bespielsweise HIV.

Die erfindungsgemäße Verwendung des markierten Liganden als Diagnostikum kann auch der Bestimmung von Verteilungs- und/oder Wanderungsmustern von CD4-tragenden Zellen bei Anwesenheit eines oder mehrere Transplantate in einem menschlichen Individuum, wie Zellen, Gewebe oder Organe und/oder Implantate, wie Gewebs- oder Gefäßersatz, Depots, Pumpen oder Filtern, dienen.

Die Bestimmung der Wanderungs- und/oder Verteilungsmuster wird vorzugsweise durch medizinisch-bildgebende Verfahren durchgeführt, wie Radioimaging, Magnetresonanzverfahren oder gegebenenfalls computergestützte tomographische bildgebende Verfahren. Die Bildgebung kann entweder aus Einzel- oder seriellen Aufnahmen bestehen. Sie kann durch eine vollständige Aufnahme oder durch teilweise Aufnahmen des Individuums erfolgen (Scanning).

Das Wanderungs- und/oder Verteilungsmuster eines Liganden der mit einem Gammastrahler markiert ist, kann mit einer Gammakamera oder einer Kamera für Einzelphotonenemissionstomografie (single photon emission computed tomography, SPECT) bestimmt werden. Positronenemitter können z.B. durch Positronenemissionstomografie (positron emission tomography, PET) bestimmt werden. Die Wanderungs- und/oder Verteilungsmuster magnetischer Partikel im Organismus können durch Magnetresonanztomografie (MRT) oder "magnetic resonance imaging" (MRI) bestimmt werden. Die Anwendung der und der Umgang mit diesen bildgebenden medizinischen Verfahren sind bekannt.

Vorzugsweise ist das medizinisch-bildgebende Verfahren eine Ganzkörper-Analyse (Scan). Die Erfindung schließt auch Scans einzelner Körperteile oder Organe ein. Die Scans können Minuten, Stunden, Tage, Wochen oder länger nach Verabreichung des markierten Liganden durchgeführt werden. Der genaue Zeitpunkt hängt von verschiedenen Faktoren ab, z.B. von Art und Menge des markierten Liganden, vom Verhalten der human-CD4-tragenden Zellen oder Partikel oder von Krankheitsverläufen. Der genaue Zeitfaktor kann mit Hilfe von üblicherweise angewendeten Optimierungsalgorithmen durch erfahrenes Fachpersonal ermittelt werden. Das medizinisch-bildgebende Verfahren wird mit einzelnen oder mit seriellen Scans abgeschlossen.

Durch die erfindungsgemäße Verwendung des markierten Liganden als Diagnostikum zur Analyse von Wanderungs- und/oder Verteilungsmustern von CD4-tragenden Zellen kann weiterhin die therapeutische Antwort oder die Therapieeffizienz in einem menschlichen Individuum, das an einer oder mehreren der besagten Erkrankungen leidet, bestimmt werden. Das Individuum wird mit einer Therapie für die entsprechende(n) Erkrankung(en) behandelt. Das Wanderungs- und/oder Verteilungsmuster von CD4-tragenden Zellen in dem behandelten Individuum als Reaktion auf die Therapie wird mit medizinisch-bildgebenden Verfahren bestimmt. Dies erfolgt derart, dass festgestellt wird, ob die Therapie die Verteilungs- und/oder Wanderungsmuster human-CD4-exprimierender Zellen verändert.

Durch die erfindungsgemäße Verwendung des markierten Liganden als Diagnostikum zur Analyse von Wanderungs- und/oder Verteilungsmustern von CD4-tragenden Zellen kann auch das Potenzial einer Verbindung oder eines Arzneimittels zur Veränderung der Verteilungs- und/oder Wanderungsmuster human-CD4-exprimierender Zellen im menschlichen Organismus abgeschätzt werden, indem mit Hilfe medizinisch-bildgebender Verfahren, wie vorstehend beschrieben, bestimmt wird, ob die Verbindung oder das Arzneimittel die Verteilungs- und/oder Wanderungsmuster der human-CD4-exprimierenden Zellen im menschlichen Organismus verändert. Die Verteilungs- und/oder Wanderungsmuster sind dann verändert, wenn sie von einem standardisierten Verteilungs- und/oder Wanderungsmuster abweichen.

Die erfindungsgemäße Verwendung des markierten Liganden als Diagnostikum zur Analyse von Wanderungs- und/oder Verteilungsmustern CD4-tragender Zellen kann auch zur Identifizierung von Arzneistoffen für die Behandlung von Autoimmunerkankungen, Tumorerkrankungen oder Infektionskrankheiten dienen, indem bestimmt wird, ob die Verbindung oder das Arzneimittel die Verteilungs- und/oder Wanderungsmuster von human-CD4-exprimierenden Zellen in dem menschlichen Organismus verändert. Die durch die Verabreichung der Verbindung oder des Arzneimittels erhaltenen Verteilungs- und/oder Wanderungsmuster werden mit denen in menschlichen Individuen, die nicht an einer der genannten Erkrankungen leiden, verglichen.

Die erfindungsgemäße Verwendung des markierten Liganden als Diagnostikum zur Analyse von Wanderungs- und/oder Verteilungsmustern CD4-tragender Zellen erlaubt auch die Bestimmung der optimalen Führung einer immunsupressiven Therapie nach Transplantation nicht-autologer oder aus anderen Gründen für den Empfänger potenziell immunogener Zellen, Gewebe oder Organe sowie nach Implantation von synthetischen und halbsynthetischen Materialien zum Ersatz oder zur Unterstützung von Geweben und Organen und deren Funktionen, indem mit Hilfe medizinisch-bildgebender Verfahren, wie vorstehend beschrieben, bestimmt wird, ob sich die Verteilungs- und/oder Wanderungsmuster human-CD4-tragender Zellen derart verändern, dass auf eine mit dem Transplantat/Implantat assoziierte entzündliche Reaktion bzw. Immunreaktion geschlossen werden kann. Dieser Schluss kann entweder durch Abweichen der besagten Verteilungs-und/oder Wanderungsmuster von standardisierten Mustern oder durch Abweichen von Mustern, die zu einem früheren Zeitpunkt an besagtem Individuum bestimmt wurden, erfolgen.

Ein weiterer Gegenstand der vorliegenden Erfindung ist eine Zusammensetzung, die einen markierten Liganden mit Spezifität für das CD4-Molekül ausgewählt aus der Gruppe bestehend aus Antikörperfragmenten ausgewählt aus F(ab')2, Fab' und Fab des Antikörpers Max.16H5 und rekombinanten Antikörperfragmenten ausgewählt aus F(ab')2, Fab' und Fab des Antikörpers Max.16H5 und CD4-tragende Zellen oder Partikel umfasst. Vorzugsweise sind die CD4-tragenden Zellen oder Partikel human-CD4-tragende Zellen oder Partikel.

Vorzugsweise ist die Zusammensetzung ein therapeutisches oder diagnostisches Mittel, besonders bevorzugt ist die Zusammensetzung ein Diagnostikum zur Bestimmung der Verteilungs- und Wanderungsmuster CD4-tragender Zellen oder Partikel in einem Individuum, vorzugsweise in einem menschlichen Individuum.

Der markierte Ligand und die CD4-tragenden Zellen, sind wie vorstehend beschrieben. Die Partikel sind vorzugsweise ausgewählt aus sphärischen Partikeln verschiedener Materialien. Die Partikel können beispielsweise Liposomen sein. Die CD4-tragenden Partikel können mit komplementären Strukturen, z.B. auf Körperzellen, Bakterien oder Viren, interagieren, dieselben markieren oder Wirkstoffe in deren Nähe transportieren. Die Partikel können auch einen Arzneistoff enthalten, vorzugsweise ein Diagnostikum oder ein Therapeutikum.

Die erfindungsgemäße Zusammensetzung, insbesondere das Diagnostikum, ist geeignet zur Bestimmung der Migration von human-CD4-exprimierenden Zellen bzw. synthetischen Partikeln in menschlichen Individuen. Zu diesem Zweck werden CD4-tragende Zellen, wie z.B. Entzündungszellen, oder Partikel mit den markierten Liganden derart inkubiert, dass eine erfindungsgemäße Zusammensetzung, umfassend markierte Zellen oder Partikel, erhalten wird. Diese Zusammensetzung, insbesondere das Diagnostikum, umfassend markierte Zellen oder Partikel, kann einem Patienten verabreicht und deren Verteilungs- und Wanderungsmuster durch medizinisch-bildgebende Verfahren, wie vorstehend beschrieben, bestimmt werden.

Die erfindungsgemäße Zusammensetzung, insbesondere das Diagnostikum, kann durch in Kontakt bringen des markierten Liganden ausgewählt aus der Gruppe bestehend aus Antikörperfragmenten ausgewählt aus F(ab')2, Fab' und Fab des Antikörpers Max.16H5 und rekombinanten Antikörperfragmenten ausgewählt aus F(ab')2, Fab' und Fab des Antikörpers Max.16H5 mit den CD4-tragenden Zellen und/oder Partikeln hergestellt werden. Die CD4-tragenden Zellen können entweder von dem selben menschlichen Individuum stammen, in dem die Wanderungs- und/oder Verteilungsmuster der CD4-tragenden Zellen bestimmt werden sollen, oder von einem anderen menschlichen Individuum. Die Herstellung der Zusammensetzung, insbesondere des Diagnostikums, kann in vitro derartig durchgeführt werden, dass ein markierter Ligand spezifisch mit CD4-tragenden Zellen oder Partikeln interagiert, so dass markierte human-CD4-tragende Zellen oder Partikel entstehen. Wenn die erfindungsgemäße Zusammensetzung CD4-tragende Zellen umfasst, müssen vorteilhafterweise die human-CD4-tragenden Zellen zur spezifischen Markierung mit dem Liganden nicht separiert oder isoliert werden. Sie können beispielsweise in einer Zellpopulation vorliegen. Eine solche Zellpopulation kann zur Markierung der human-CD4-tragenden Zellen mit dem markierten Liganden inkubiert werden. Beispielsweise kann Peripherblut mit dem markierten Liganden inkubiert werden, wodurch ein erfindungsgemäßes Diagnostikum erhalten wird. Der markierte Ligand interagiert mit den human-CD4-tragenden Zellen oder Partikeln, sodass spezifisch markierte Zellen oder Partikel entstehen. Interagieren bedeutet dabei z.B. binden, assoziieren, komplexieren oder konjugieren.

Die Verabreichung des markierten Liganden in ein menschliches Individuum kann mit allen Methoden durchgeführt werden, die dessen spezifische Interaktion mit human-CD4-tragenden Zellen oder Partikeln im menschlichen Organismus ermöglichen, wobei markierte human-CD4-tragende Zellen oder Partikel entstehen oder gebildet werden. Die Methoden umfassen Injektion, Infusion, Einbringen in Depots, Implantation, orale Ingestion und rektale Verabreichung sowie die lokale Applikation. Die bevorzugte Verabreichungsmethode ist die Injektion, die z.B. intravenös, intradermal, subkutan, intramuskulär oder intraperitoneal erfolgen kann. In bestimmten Fällen werden mehrfache oder kombinierte Verabreichungsmethoden angewandt. Der markierte Ligand kann in gelöster oder kolloidaler Form vorliegen, wobei die Trägerflüssigkeiten für eine Verabreichung in menschliche Organismen geeignet sind, wie z.B. Wasser oder physiologische Salzlösung für eine Injektion.

Das erfindungsgemäße Diagnostikum kann in einer Dosis verwendet werden, die vorzugsweise zwischen 0,01 mg/kg und 7 mg/kg Körpergewicht beträgt. Falls der Ligand ein Antikörper ist, liegt die Dosis vorzugsweise zwischen 0,2 mg/Untersuchung und 7 mg/Untersuchung bzw. zwischen 0,003 mg/kg und 0,1 mg/kg Körpergewicht. Die Menge der notwendigen Radioaktivität wird durch das angewendete Isotop bestimmt und kann von erfahrenem Fachpersonal bestimmt werden. Wenn der Ligand mit einem radioaktiven Isotop markiert ist, kann die verwendete Aktivität zwischen 1,5 und 10,0 mCi je Einzeldosis bei Indium-111, zwischen 10 und 30 mCi je Einzeldosis bei Technetium-99, zwischen 5 und 10 mCi je Einzeldosis bei Jod-123, zwischen 5 und 10 mCi je Einzeldosis bei Jod-124, zwischen 10 und 20 mCi je Einzeldosis bei Fluor-18 und zwischen 20 und 30 mCi je Einzeldosis bei Kohlenstoff-11 liegen.

Wenn das Diagnostikum human-CD4-tragende Partikel umfasst, kann dieses in vitro derartig hergestellt werden, dass ein markierte Ligand, wie vorstehend beschrieben, spezifisch mit den Partikeln interagieren kann, so dass ein erfindungsgemäßes Diagnostikum, umfassend markierte human-CD4-tragende Partikel, erhalten wird.

Die Verabreichung der markierten human-CD4-tragenden Partikel in den menschlichen Organismus kann durch verschiedene Methoden erfolgen, wie vorstehend in bezug auf das erfindungsgemäße Diagnostikum, umfassend CD4-tragenden Zellen und markierte Liganden, beschrieben.

Die Bestimmung der Wanderungs- und Verteilungsmuster der erfindungsgemäßen Zusammensetzung, insbesondere des erfindungsgemäßen Diagnostikums, ist auf den selben Anwendungsgebieten vorteilhaft einsetzbar, wie vorstehend in Bezug auf die erfindungsgemäße Verwendung der markierten Liganden mit Spezifität für das humane CD4-Molekül beschrieben.

In einem weiteren Aspekt betrifft die vorliegende Offenbarung ein Verfahren zur Bestimmung des Ausmaßes und der Progredienz von Erkrankungen, bei denen human-CD4-tragende Zellen von klinischer Bedeutung sind, die Schritte umfassend
a) Bereitstellen einer in-vivo-Analyse des Verteilungs- und/oder Wanderungsmusters von human-CD4-tragenden Zellen in einem Individuum,
b) Bereitstellen einer Standard-in-vivo-Analyse des Verteilungs- und/oder Wanderungsmusters von human-CD4-tragenden Zellen in einem Individuum, und
c) Bestimmen der Abweichungen der in Schritt a) und Schritt b) bereitgestellten Analysen, wodurch das Ausmaß und die Progredienz der Erkrankung bestimmt wird.

Die in Schritt a) und/oder Schritt b) bereitgestellte Analyse ist vorzugsweise erhältlich durch die Verabreichung eines markierten Liganden oder des erfindungsgemäßen Diagnostikums in ein Individuum, wie vorstehend beschrieben und Bestimmen des Verteilungs-und/oder Wanderungsmusters von CD4-tragenden Zellen oder des Diagnostikums in dem Individuum mit medizinsch-bildgebenden Verfahren, wie ebenfalls vorstehend beschrieben.

Die in Schritt a) bereitgestellte Analyse ist vorzugsweise von einem erkrankten, menschlichen Individuum. Die Erkrankung ist vorzugsweise ausgewählt aus der Gruppe, bestehend aus Autoimmunerkrankungen, Tumorerkrankungen, Infektionserkrankungen und Abstoßungskrisen nach Transplantationen, wie vorstehend beschrieben.

Die Standard-Analyse in Schritt b) ist bevorzugt die Analyse des Verteilungs- und/oder Wanderungsmusters von human-CD4-tragenden Zellen in einem nicht erkrankten Individuum.

Die Standard-Analyse in Schritt b) ist bevorzugt ein Verteilungs- und/oder Wanderungsmuster eines Individuums, das aus einer vorangegangenen Analyse desselben menschlichen Individuums erhalten wird.

In Schritt a) kann auch die Analyse des Verteilungs- und/oder Wanderungsmusters von human-CD4-tragenden Zellen in einem Individuum bereitgestellt werden, das zusätzlich einer Therapie gegen die Erkrankung unterzogen wurde. Die Therapie besteht vorzugsweise in der Verabreichung einer Verbindung oder eines Arzneimittels. Die Antwort des Individuums auf die Therapie wird dadurch charakterisiert, dass mit medizinisch-bitdgebenden Verfahren bestimmt wird, ob die Therapie die Verteilungs- und/oder Wanderungsmuster von human-CD4-tragenden Zellen verändert. Vorzugsweise werden diese Muster mit solchen des erkrankten Individuums, beispielsweise vor Therapiebeginn oder zu einem früheren Therapiezeitpunkt oder mit denen von nicht erkrankten Individuen verglichen.

Nachstehend wird die Erfindung anhand von Figuren und Beispielen verdeutlicht.

### Figuren

**Figur 1** zeigt Dot-Blots einer spezifischen Anfärbung von murinen und humanen Helfer-T-Lymphozyten. Die Dot-Blots repräsentieren folgende Doppelfärbungen: **A.** Mauszellen: murines CD3/murines CD4 (GK 1,5); **B.** Menschliche Zellen: humanes CD3/humanes CD4 (Max. 16H5); **C.** Mauszellen: murines CD3/humanes CD4 (Max. 16H5); **D.** Menschliche Zellen: humanes CD3/murines CD4 (CK 1.5).

**Figur 2** zeigt die Darstellung des Konformationsisotops des humanen CD4 im Kalottenmodell.

### Beispiele

### Beispiel 1: Produktion von Anti-human-CD4 und enzymatische Spaltung zu Fab'-Fragmenten.

Das Beispiel beschreibt die Produktion und Reinigung eines monoklonalen Maus-Antikörpers mit Spezifität für humanes CD4 sowie dessen enzymatische Spaltung in Fab'-Fragmente.

Der monoklonale Antikörper Max.16H5 erkennt ein Epitop in der D1-Domäne des humanen CD4. Das Immunglobulin (IgG1) wurde aus Hybridomüberständen über Protein-A-Affinitätschromatografie isoliert. Das Eluat aus der Protein A-Säule wurde neutralisiert, gegen PBS dialysiert und die Immunglobulinkonzentration auf 1 mg/ml eingestellt. Identität und Spezifität des monoklonalen Antikörpers wurden mittels SDS-Gelelektrophorese unter Verwendung von Maus-IgG1 als Kontrolle, und im Western Blot mit rekombinantem humanem CD4 nachgewiesen. Die Bindung an nativ exprimiertes humanes CD4 (Bioaktivität) wurde mittels Durchflußzytometrie (FACS) unter Verwendung eines FACScan (Becton-Dickinson) nachgewiesen. Als Indikatorzellen dienten kryokonservierte humane periphere mononukleäre Zellen (PBMZ), die einen (individuell verschiedenen) Anteil an human-CD4-exprimierenden Helfer-T-Lymphozyten enthalten. Diese wurden nach Auftauen mit Anti-human-CD4 inkubiert, gewaschen, und gebundenes Maus-IgG wurde mit Fluoreszenz(FITC)-markierten Ziege-anti-Maus-Immunglobulin-Fab2-Fragmenten (DAKO F 0479) im FACScan quantitativ bestimmt.

Anti-CD4-Gesamt-IgG wurde mit Papain zu Fab'- und Fc-Fragmenten verdaut. Die enzymatische Spaltung wurde entsprechend zuvor optimierter Reaktionszeiten abgebrochen und das Reaktionsgemisch wurde mittels Säulenchromatographie an Protein-A fraktioniert. Fc-Fragmente werden dabei an Protein-A gebunden und im Eluat erscheinen vorwiegend Fab'-Fragmente. Größe (ca. 50 kD) und Anteil (ca 90%) der eluierten Fab'-Fraktion wurden mittels SDS-Gelelektrophorese bestimmt. Die Bioaktivität der Anti-human-CD4-Fab'-Fraktion, d.h. dessen Vermögen selektiv an nativ exprimiertes humanes CD4 zu binden, wurde durch FACS-Analysen quantitativ bestimmt. Dazu wurden humane PBMZ nacheinander mit Fab'-Fragmenten und FITC-markiertem Ziege-Anti-Maus-Immunglobulin inkubiert und die human-CD4-exprimierenden Zellen im Flusszytometer analysiert.

### Beispiel 2: Radiochemische Markierung von Anti-human-CD4-Fab'-Fragmenten mit 111-In.

Das Beispiel beschreibt die Derivatisierung von Anti-human-CD4- Fab'-Fragmenten mit einem Chelatbildner.

Anti-human-CD4-Fab' wird in 0,1 M NaHCO₃ Puffer über Anhydridreaktion mit zyklischer Diethylentriamin-Pentaessigsäure (cDTPA) umgesetzt. Das Fab'-DTPA-Derivat wird dann in 0,5 M Natriumacetatpuffer pH 5,4 überführt, über ein 10 kD Ausschlussfilter angereichert und über Größenausschlußchromatografie gereinigt. Aliquots der derivatisierten Fragmente werden auf eine Proteinkonzentration zwischen 0,5 und 1 mg/ml eingestellt und bis zur radiochemischen Markierung bei +4 °C gelagert.

Das Radiodiagnostikum wird durch Markierung des Anti-human-CD4-Fab'-DTPA-Derivates mit 111-Indium (Halbwertszeit: 2,6 Tage, mittelenergetische Gamma-Strahlung von ca. 247 keV) unmittelbar vor Injektion präpariert. Dazu werden etwa 10 g DTPA-derivatisiertes Fragment mit 300 Ci 111-Indiumchlorid für 30 min bei Raumtemperatur inkubiert. Freies Indiumchlorid wird über PD-10-Säulen vom Radiodiagnostikum (111-Indium-markiertes Anti-human-CD4-Fab') abgetrennt.

### Beipiel 3: Bindungsassay mit Anti-CD4-Fab'-DTPA

Das Beispiel weist nach, dass sich Spezifität und Bioaktivität von Anti-CD4-Fab' aus Beispiel 1 und DTPA-konjugiertem Anti-CD4-Fab'-DTPA aus Beispiel 2 nicht unterscheiden.

Indikatorzellen wurden mit stufenweise steigenden Konzentrationen des Fab' bzw. des DTPA-derivatisierten Fab' des Anti-human-CD4 inkubiert und nicht gebundene Liganden wurden mit phosphatgepufferter Natriumchloridlösung (PBS) ausgewaschen. Die an den Indikatorzellen (humane PBMZ) gebundenen Liganden wurden mit Fluoreszenz(FITC)-markierten Ziege-anti-Maus-lmmunglobulin-Fab2-Fragmenten (DAKO F0479) inkubiert, wiederum gewaschen und die Fluoreszenz human-CD4-exprimierender Zellen wurde im FACS quantitativ ermittelt.

Die Spezifität der Anti-CD4-Fab'-Fragmente (für humanes CD4) wird durch die Derivatisierung mit DTPA nicht beeinflusst. DTPA-derivatisierte und nicht-derivatisierte Anti-CD4-Fab'-Fragmente identifizieren gleich große Fraktionen fluoreszierender human-CD4-tragende Indikatorzellen. Die biologische Aktivität beider Liganden war dabei vergleichbar, eine Sättigung für die Fluoreszenzfärbung wurde bei gleichen Konzentrationen von Fab' bzw. Fab'-DTPA erreicht. Ebenso war der Fluoreszenzverlust bei sinkenden Konzentration der Liganden vergleichbar.

### Beispiel 4: Bioverteilung von 111-Indium-markierten Anti-human-CD4-Fab'-Fragmenten.

Das Beispiel zeigt die selektive Anreicherung von 111-Indium-markiertem Anti-human-CD4-Fab-DTPA (Radiodiagnostikum) in Geweben mit hoher human-CD4-Expression.

Als präklinisches Modell zur in vivo-Untersuchung der Bioverteilung des Radiodiagnostikums kam ein Mausmodell mit transgener Expression des humanen CD4 zur Anwendung (CD4/DR3-Maus). Anstelle des eigenen ("Wildtyp"-)CD4 wird im CD4/DR3 Mausmodell humanes CD4 exprimiert, wobei die Expressionsmuster für humanes CD4 der natürlichen Verteilung des Rezeptors korrekt entsprechen (Laub 2001).

CD4/DR3-Mäusen und Wildtyp-Mäusen (C57/BL6) wurden jeweils 20 Ci des Radiodiagnostikums intravenös injiziert: 1, 4, 24 und 48 Stunden nach Injektion wurden lymphatische und nicht-lymphatische Organe entnommen, gewogen und die inkorporierte Radioaktivität mit einem Gamma-Counter gemessen (LKB model 1282, Wallac-Perkin Elmer, Turku Finland). Zur Korrektur der Halbwertszeit wurden Aliquots des Radiodiagnostikums zurückbehalten und simultan mit den Proben vermessen. Für die Analyse der Bioverteilung wurden die radioaktiven Signale (Counts) in % der injizierten Aktivität je Gramm Gewebe umgerechnet.

In CD4/DR3-Mäusen wurden signifikant erhöhte Aktivitäten in Lymphknoten, Milz und Knochenmark gemessen. In nicht-lymphatischen Organen (Leber, Muskel, Lunge und Herz) erfolgte keine Anreicherung des 111-Indium-markierten Anti-human-CD4 Fab'-Fragmentes (Laub 2000). In den Nieren fanden sich Signale, die auf die renale Elimination des Radiodiagnostikums hinweisen.

Die Untersuchungen zur Bioverteilung zeigen, dass die Verteilung des Radiodiagnostikums in lymphatischem Gewebe der CD4/DR3-Maus auf spezifischen Interaktionen zwischen dem transgen exprimierten humanen CD4 und dem 111-Indium-makierten Anti-human-CD4-Fab' beruht.

### Beispiel 5: Abhängigkeit der spezifischen Anreicherung von der Expression des humanen CD4.

Mit diesem Beispiel wird nachgewiesen, dass spezifische Signale des Radiodiagnostikums die Anwesenheit von humanem CD4 erfordern.

In Balb/c-Mäuse (Wildtyp) mit normaler Expression des Maus-CD4 wurden 20 Ci des Radiodiagnostikums aus Beispiel 2 intravenös injiziert. Nach 1, 4, 24 und 48 Stunden wurden von je drei Balb/c-Mäusen Proben wie in Beispiel 4 entnommen und die inkorporierte Radioaktivität gemessen. Nur in den Nieren wurden zu allen Zeiten erhöhte Signale ermittelt, was auf die renale Elimination des Radiodiagnostikums bzw. des Radionuklides verweist. Obwohl lymphatische Organe wie Milz, Lymphknoten, und Knochenmark bis 20 % Maus-CD4-exprimierende Zellen enthalten, waren hier keine Signale erfassbar. Die Ergebnisse zeigen, dass das 111-In-markierte Anti-human-CD4-Fab' spezifisch humanes CD4 bindet und nicht in lymphatischen Organen der Maus unspezifisch angereichert wird.

### Beispiel 6: In vivo-Lokalisierung von Helfer-T-Lymphozyten in einem Mausmodell mit transgener Expression des humanen CD4.

Das Beispiel beweist dass mit dem Radiodiagnostikum Organe und Gewebe mit hohem Anteil an human-CD4-exprimierenden Zellen in-vivo lokalisiert werden können.

Jeweils 20 Ci des Radiodiagnostikums wurden in die Schwanzvene von Wildtyp-Mäusen und transgenen CD4/DR3-Mäusen, die das humane CD4 anstelle des Wildtyp CD4 exprimieren, injiziert. 1, 24 und 48 Stunden post injektionem wurden die Tiere narkotisiert und in Bauchlage im Messbereich einer Gamma-Kamera (Siemens Icon) positioniert. Mit Hilfe eines planaren Kollimators für mittelenergetische Gamma-Strahlung wurden über 30 min die Signale der Gamma-Photonen aufgezeichnet und zu planaren Ganzkörperszintigrammen zusammengesetzt. Die weitere Auswertung der Szintigramme erfolgte mit einer Software für medizinisch-bildgebenden Verfahren (Osiris, Universitätshospital Genf, Schweiz).

Eine Stunde nach Injektion des Radiodiagnostikums werden in human-CD4-tragenden und Wildtypmäusen besonders starke Signale in den Nieren nachgewiesen. In Wildtypmäusen persistiert ein wenig differenzierbarer Hintergrund und die Nierensignale bleiben über den gesamten Messzeitraum bestehen, was auf die renale Elimination des Radiodiagnostikums verweist, ohne dass es zu spezifischen Anreicherungen in anderen Organen kommt. In CD4/DR3-Mäusen mit transgener Expression des humanen CD4 finden sich vier Stunden nach Injektion neben den Nierensignalen auch solche im Bereich der Milz. Deutliche Signale werden weiterhin im Bereich der vorderen und hinteren Extremitäten gemessen, die von human-CD4 exprimierenden Zellen im Knochenmark stammen (z.B. Helfer-T-Lymphozyten, eosinophile Granulozyten). Die Hintergrundsignale sind stärker strukturiert und verweisen auf Kompartimente des hämatopoetischen und lymphatischen Systems, ohne dass es zu einer Darstellung distinkter Lymphknoten oder Gefäße kommt, was mit der geringen Auflösung bei 111-Indium-Szintigrammen (ca. 4 mm) erklärbar ist. Die Szintigramme von CD4/DR3-Mäusen 24 und 48 Stunden nach Injektion des Radiodiagnostikums zeigen einen deutlichen Rückgang der Nierensignale, woraus eine verbesserte Milzdarstellung resultiert. Die Ergebnisse verweisen auf hohe Anteile human-CD4-exprimierender Zellen. FACS-Analysen an CD4/DR3-Mäusen bestätigen hohe Anteile an human-CD4-exprimierenden Helfer-T-Lymphozyten vor allem in der Milz [Laub 2001, JIM].

Die differenzierten Signale verweisen auf die selektive Anreicherung des Radiodiagnostikums in Organen mit human-CD4-exprimierenden Zellen. Diese Anreicherung ist spezifisch, da sie in Wildtyp-Mäusen ohne transgenes humanes CD4 als Zielmolekül nicht auftritt und wird über die spezifische Interaktion zwischen humanem CD4 und 111-Indium-markierten Anti-human-CD4-Fab'-Fragmenten bestimmt.

### Beispiel 7: Spezifische Anfärbung von murinen und humanen Helfer-T-Lymphozyten

Mit diesem Beispiel wird nachgewiesen, dass monoklonale Antikörper gegen humanes bzw. murines CD4 nicht miteinander kreuzreagieren.

Peripherblut von Mensch und Maus wurde mit Fluoresceinisothiocyanat(FITC)-markierten monoklonalen Antikörpern gegen murines CD4 (Klon GK 1.5; vgl. US 6,146,614) und humanes CD4 (Klon Max.16H5) inkubiert. Zur Detektion der T-Lymphozyten wurden die Zellsuspensionen zusätzlich mit Phycoerythrin (PE)-markierten Antikörpern gegen murines bzw. humanes CD3 inkubiert. Nach einer Inkubationszeit von 30 min wurden die Zellsuspensionen gewaschen und flusszytometrisch (FACScan, Becton Dickinson) analysiert.

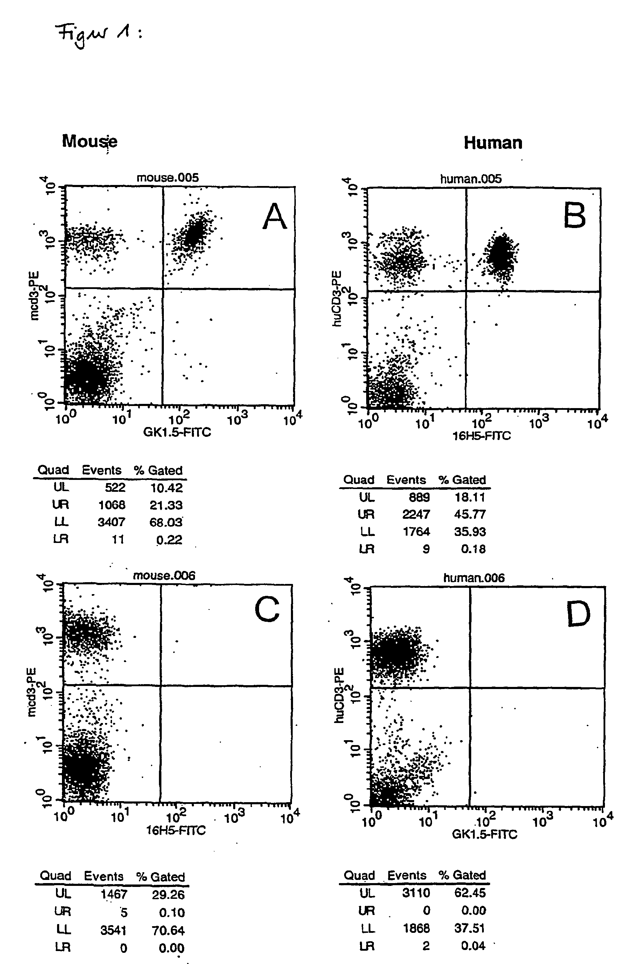

In murinen Zellsuspensionen wurden weder CD3-positive noch CD3-negative Zellen aufgefunden, an die auch Max.16H5 bindet (Quadranten UR und UL in Blot C). Umgekehrt fanden sich unter den humanen Zellen keine Zellen, die GK 1.5 binden (Quadrandten UR und UL in Blot D). Die Färbungen in Figur 1 zeigen somit, dass GK 1.5 nur an murine Helfer-T-Lymphozyten (Blot A) und Max.16H5 nur an humane Helfer-T-Lymphozyten (Blot B) bindet.

### Beispiel 8: Analyse der Bindungsstelle von Max.16H5 (Anti-human-CD4) am menschlichen CD4 (Epitopmapping)

Dieses Beispiel zeigt die Identifizierung der Max.16H5-Bindungsstelle an der extrazellulären Region des humanen CD4.

Dazu wurden mit Hilfe eines Auto-Spot Robot (ASP222) 177 Peptide aus der linearen Aminosäuresequenz der Domänen 1 bis 4 (D1-D4) des humanen CD4-Moleküls an punktförmigen Arealen (Spot) einer mit PEG-Spacern derivatisierten Membran synthetisiert. Die Peptidsequenzen in den Spots waren 12 Aminosäuren lang und enthielten die jeweils 10 letzten Aminosäuren der vorhergehenden Sequenz (Raster: 12-mere mit 10-er Overlap).

Diese Membranen wurden mit 2 % Milchpulver geblockt und mit 10 µg/ml Max.16H5 für 120 min bei Raumtemperatur inkubiert. Danach wurden die Membranen gewaschen und mit einem Peroxidase-markierten Ziege-anti-Maus-Antikörper (ZAM-POD, Dianova) für 75 min bei Raumtemperatur inkubiert. Nach einem weiteren Waschschritt erfolgte die Detektion von ZAM-POD mit Hilfe eines Chemolumineszenzsubstrates (Super Signal Wes Dura, Pierce). Die Chemolumineszenz-Signale auf der Membran wurden mit Hilfe eines Lumineszenz-Image Readers (Fujifilm LAS-1000) detektiert und digitalisiert.

Für die Bindung von Max.16H5 an den gespotteten Peptiden waren die folgende Aminosäuresequenz verantwortlich:

| | | | | | | |
|---|---|---|---|---|---|---|
| p34: | GDT | VEL | TCT | ASQ | | (SEQ ID NO. 1) |
| p46: | KKS | IQF | HWK | K | | (SEQ ID NO. 2) |
| p115: | KEE | VQL | LVF | | | (SEQ ID NO. 3) |
| p151: | PSV | QCR | SPR | | | (SEQ ID NO. 4) |
| p223: | FPL | AFT | VEK | LTG | SGE | (SEQ ID NO. 5) |
| p307: | KLII | QEV | NLV | VMR | | (SEQ ID NO. 6) |

Aus den positiven Spots wurden die für die Bindung von Max.16H5 verwendeten Aminosäuresequenzen an einem dreidimensionalem Modell des humanen CD4 rekonstruiert. Dabei zeigte sich, dass Max.16H5 mit einer räumlich strukturierten Bindungsstelle (Konformationsepitop) interagiert.

## Patentansprüche

1. Verwendung eines markierten Liganden mit Spezifität für das humane CD4-Molekül ausgewählt aus der Gruppe bestehend aus Antikörperfragmenten ausgewählt aus F(ab')₂, Fab' und Fab des Antikörpers Max.16H5 und rekombinanten Antikörperfragmenten ausgewählt aus F(ab')₂, Fab' und Fab-Fragmenten des Antikörpers Max.16H5 zur Herstellung eines in vivo Diagnostikums zur Analyse von Wanderungs- und/oder Verteilungsmustern von Zellpopulationen, die human-CD4-tragende Zellen umfassen, in menschlichen Individuen.

2. Verwendung nach Anspruch 1, wobei die Antikörperfragmente oder die rekombinanten Antikörperfragmente monoklonale Antikörperfragmente sind oder davon abgeleitet sind.

3. Verwendung nach Anspruch 1 oder 2, wobei die Antikörperfragmente oder die rekombinanten Antikörperfragmente eine der in SEQ ID NO. 1 bis SEQ ID NO. 6 gezeigten Sequenzen erkennen und daran binden.

4. Verwendung nach einem der Ansprüche 1 bis 3, wobei die Antikörperfragmente oder die rekombinanten Antikörperfragmente das Konformationsepitop des humanen CD4 erkennen und daran binden.

5. Verwendung nach einem der vorstehenden Ansprüche, wobei die Markierung des Liganden ausgewählt wird aus der Gruppe bestehend aus Gamma-Emittern, Positronen-Emittern, magnetischem Material, Dichtekontrastmaterial und Mischungen davon.

6. Verwendung nach Anspruch 5, wobei die Markierung des Liganden ein Gamma-Emitter ist, der ein oder mehrere Radioisotope, ausgewählt aus der Gruppe bestehend aus Indium-111, Technetium-99m, Technetium-99, Jod-132, anderen Radioisotopen für medizinisch bildgebende Verfahren und deren Mischungen, umfasst.

7. Verwendung nach Anspruch 6, wobei das Radioisotop Technetium-99m oder Indium-111 ist.

8. Verwendung nach Anspruch 5, wobei die Markierung des Liganden ein Positronen-Emitter ist, der ein oder mehrere Isotope, ausgewählt aus der Gruppe bestehend aus Fluor-18, Kohlenstoff-11, Jod-124, anderen Isotopen für medizinisch-bildgebende Verfahren und deren Mischungen, umfasst.

9. Verwendung nach Anspruch 5, wobei die Markierung des Liganden ein magnetisches Material umfasst, ausgewählt aus der Gruppe bestehend aus Gadolinium, superparamagnetischen Substanzen, hydratisierten Eisenoxidpartikeln, anderen Materialien für medizinisch-bildgebende Verfahren und deren Mischungen.

10. Verwendung nach Anspruch 5, wobei der markierte Ligand ein Dichtekontrastmaterial umfasst.

11. Verwendung nach einem der vorstehenden Ansprüche, wobei die human-CD4-tragenden Zellen ausgewählt sind aus der Gruppe bestehend aus T- und B-Lymphozyten, Monozyten, Makrophagen, dendritischen und Langerhans-Zellen und eosinophilen Granulozyten.

12. Verwendung nach Anspruch 11, wobei die Lymphozyten aus einer Gruppe oder Population ausgewählt werden, die nicht-lymphozytäre Zellen umfasst.

13. Verwendung nach einem der vorstehenden Ansprüche, wobei die Zellpopulation von einem menschlichen Individuum stammt.

14. Zusammensetzung, umfassend einen markierten Liganden mit Spezifität für das CD4-Molekül, ausgewählt aus der Gruppe bestehend aus Antikörperfragmenten, ausgewählt aus F(ab')₂, Fab' und Fab des Antikörpers Max.16H5 und rekombinanten Antikörperfragmenten, ausgewählt aus F(ab')₂, Fab' und Fab des Antikörpers Max.16H5, und CD4-tragende Zellen oder Partikel.

15. Zusammensetzung nach Anspruch 14, wobei die CD4-tragenden Zellen oder Partikel human-CD4-tragende Zellen oder Partikel sind.

16. Zusammensetzung nach Anspruch 14 oder 15 als Diagnostikum.

17. Verwendung einer Zusammensetzung nach einem der Ansprüche 14 bis 16 zur Herstellung eines Diagnostikums zur Bestimmung der Wanderungs- und/oder Verteilungsmuster in einem Individuum.

18. Verwendung eines markierten Liganden mit Spezifität für das humane CD4-Molekül, ausgewählt aus der Gruppe bestehend aus Antikörperfragmenten, ausgewählt aus F(ab')₂, Fab' und Fab des Antikörpers Max.16H5 und rekombinanten Antikörperfragmenten, ausgewählt aus F(ab')₂, Fab' und Fab des Antikörpers Max.16H5, zur Herstellung eines in vivo Diagnostikums zur Bestimmung des Ausmaßes und der Progredienz von Erkrankungen, bei denen human-CD4-tragende Zellen von klinischer Bedeutung sind.

19. Verwendung nach Anspruch 18, wobei die Erkrankung ausgewählt ist aus der Gruppe, bestehend aus Autoimmunerkrankungen, Tumorerkrankungen, Infektionserkrankungen und Abstoßungskrisen nach Transplantationen.

## Claims

1. Use of a labelled ligand having specificity for the human CD4 molecule selected from the group comprising antibody fragments selected from F(ab')₂, Fab' and Fab of the antibody Max.16H5 and recombinant antibody fragments selected from F(ab')₂, Fab' and Fab fragments of the antibody Max.16H5 to produce an in vivo diagnostic agent for analysing migration and/or distribution patterns of cell populations which contain human CD4-bearing cells in human individuals.

2. Use according to claim 2, **characterized in that** the antibody fragments or the recombinant antibody fragments are monoclonal antibody fragments or are derived therefrom.

3. Use according to claim 1 or 2, **characterized in that** the antibody fragments or the recombinant antibody fragments recognize one of the sequences shown in SEQ ID NO. 1 to SEQ ID NO. 6 and bind thereto.

4. Use according to one of the claims 1 to 3, **characterized in that** the antibody fragments or the recombinant antibody fragments recognize the conformation epitope of human CD4 and bind thereto.

5. Use according to one of the previous claims, **characterized in that** the label of the ligand is selected from the group comprising gamma emitters, positron emitters, magnetic material, density contrast material and mixtures thereof.

6. Use according to claim 5, **characterized in that** the label of the ligand is a gamma emitter which comprises one or more radioisotopes selected from the group comprising indium-111, technetium-99m, technetium-99, iodine-132, other radioisotopes for medical imaging methods and mixtures thereof.

7. Use according to claim 6, **characterized in that** the radioisotope is technetium-99m or indium-111.

8. Use according to claim 5, **characterized in that** the label of the ligand is a positron emitter which comprises one or more isotopes selected from the group comprising fluorine-18, carbon-11, iodine-124, other isotopes for medical imaging methods and mixtures thereof.

9. Use according to claim 5, **characterized in that** the label of the ligand comprises a magnetic material selected from the group comprising gadolinium, superparamagnetic substances, hydrated iron oxide particles, other materials for medical imaging methods and mixtures thereof.

10. Use according to claim 5, **characterized in that** the labelled ligand comprises a density contrast material.

11. Use according to one of the previous claims, **characterized in that** the human CD4-bearing cells are selected from the group comprising T and B lymphocytes, monocytes, macrophages, dendritic and Langerhans cells and eosinophilic granulocytes.

12. Use according to claim 11, **characterized in that** the lymphocytes are selected from a group or population which contains non-lymphocytic cells.

13. Use according to one of the previous claims, **characterized in that** the cell population is derived from a human individual.

14. Composition containing a labelled ligand having specificity for the CD4 molecule selected from the group comprising antibody fragments selected from F(ab')₂, Fab' and Fab of the antibody Max.16H5 and recombinant antibody fragments selected from F(ab')₂, Fab' and Fab of the antibody Max.16H5, and CD4-bearing cells or particles.

15. Composition according to claim 14, **characterized in that** the CD4-bearing cells or particles are human CD4-bearing cells or particles.

16. Composition according to claim 14 or 15 as a diagnostic agent.

17. Use of a composition according to one of the claims 14 to 16 to produce a diagnostic agent for determining the migration and/or distribution pattern in an individual.

18. Use of a labelled ligand having specificity for the human CD4 molecule selected from the group comprising antibody fragments selected from F(ab')₂, Fab' and Fab of the antibody Max.16H5 and recombinant antibody fragments selected from F(ab')₂, Fab' and Fab of the antibody Max.16H5 to produce an in vivo diagnostic agent for determining the extent and progression of diseases in which human CD4-bearing cells are of clinical importance.

19. Use according to claim 18, **characterized in that** the disease is selected from the group comprising autoimmune diseases, tumour diseases, infectious diseases and rejection crises after transplantations.

## Revendications

1. Utilisation d'un ligand marqué ayant une spécificité pour la molécule CD4 humaine choisi dans le groupe constitué par des fragments d'anticorps choisis parmi F(ab')₂, Fab' et Fab de l'anticorps Max.16H5 et par des fragments d'anticorps recombinants choisis parmi des fragments F(ab')₂, Fab' et Fab de l'anticorps Max.16H5 pour la préparation d'un diagnostic *in vivo* destiné à l'analyse de profils de migration et/ou de répartition de populations cellulaires, qui comprennent des cellules porteuses de la molécule CD4 humaine, chez des individus humains.

2. Utilisation selon la revendication 1, les fragments d'anticorps ou les fragments d'anticorps recombinants étant des fragments d'anticorps monoclonaux ou dérivant de ceux-ci.

3. Utilisation selon la revendication 1 ou 2, les fragments d'anticorps ou les fragments d'anticorps recombinants reconnaissant l'une des séquences désignées dans SEQ ID NO. 1 à SEQ ID NO. 6 et se fixant à celles-ci.

4. Utilisation selon l'une des revendications 1 à 3, les fragments d'anticorps ou les fragments d'anticorps recombinés reconnaissant l'épitope conformationnel de la molécule CD4 humaine et se fixant à celui-ci.

5. Utilisation selon l'une des revendications précédentes, le marquage du ligand étant choisi dans le groupe constitué par les émetteurs gamma, les émetteurs de positrons, un matériau magnétique, un matériau à contraste de densité et des mélanges de ceux-ci.

6. Utilisation selon la revendication 5, le marquage du ligand étant un émetteur gamma qui comprend un ou plusieurs radioisotopes choisis dans le groupe constitué par l'indium-111, le technétium-99m, le technétium-99, l'iode-132, d'autres radioisotopes pour procédés d'imagerie médicale et leurs mélanges.

7. Utilisation selon la revendication 6, le radioisotope étant le technétium-99m ou l'indium-111.

8. Utilisation selon la revendication 5, le marquage du ligand étant un émetteur de positrons qui comprend un ou plusieurs isotopes choisis dans le groupe constitué par le fluor-18, le carbone-11, l'iode-124, d'autres isotopes pour procédés d'imagerie médicale et leurs mélanges.

9. Utilisation selon la revendication 5, le marquage du ligand comprenant un matériau magnétique choisi dans le groupe constitué par le gadolinium, les substances superparamagnétiques, les particules hydratées d'oxyde de fer, d'autres matériaux pour procédés d'imagerie médicale et leurs mélanges.

10. Utilisation selon la revendication 5, le ligand marqué comprenant un matériau à contraste de densité.

11. Utilisation selon l'une des revendications précédentes, les cellules porteuses de la molécule CD4 humaine étant choisies dans le groupe constitué par les lymphocytes T et B, les monocytes, les macrophages, les cellules dendritiques et les cellules de Langerhans, et les granulocytes éosinophiles.

12. Utilisation selon la revendication 11, les lymphocytes étant choisis dans un groupe ou une population qui comprend des cellules non lymphocytaires.

13. Utilisation selon l'une des revendications précédentes, la population cellulaire provenant d'un individu humain.

14. Composition comprenant un ligand marqué ayant une spécificité pour la molécule CD4 choisi dans le groupe constitué par des fragments d'anticorps choisis parmi F(ab')₂, Fab' et Fab de l'anticorps Max.16H5 et par des fragments d'anticorps recombinants choisis parmi des F(ab')₂, Fab' et Fab de l'anticorps Max.16H5, et des cellules ou des particules porteuses de la molécule CD4.

15. Composition selon la revendication 14, les cellules ou les particules porteuses de la molécule CD4 étant des cellules ou des particules porteuses de la molécule CD4 humaine.

16. Composition selon la revendication 14 ou 15 comme agent de diagnostic.

17. Utilisation d'une composition selon l'une des revendications 14 à 16, pour la préparation d'un agent de diagnostic destiné à déterminer des profils de migration et/ou de répartition chez un individu.

18. Utilisation d'un ligand marqué ayant une spécificité pour la molécule CD4 humaine choisi dans le groupe constitué par des fragments d'anticorps choisis parmi F(ab')₂, Fab' et Fab de l'anticorps Max.16H5 et par des fragments d'anticorps recombinés choisis parmi des F(ab')₂, Fab' et Fab de l'anticorps Max.16H5 pour la préparation d'un diagnostic *in vivo* destiné à déterminer l'étendue et l'évolution de maladies pour lesquelles les cellules porteuses de la molécule CD4 humaine a une signification clinique.

19. Utilisation selon la revendication 18, la maladie étant choisie dans le groupe constitué par les maladies autoimmunes, les maladies tumorales, les maladies infectieuses et les crises de rejet post-transplantation.


### SEQUENZPROTOKOLL

<110> BIOTECTID GmbH
<120> Verwendung eines markierten Liganden mit Spezifität für das humane CD4-Molekül zur Herstellung eines Diagnostikums zur Analyse von Wanderungs- und/oder Verteilungsmustern von Zellpopulationen.
<130> 24222PDE-1_DR
<140>
   <141>
<150> DE 101 60 653.2
   <151> 2001-12-11
<160> 6
<170> PatentIn Ver. 2.1
<210> 1
   <211> 12
   <212> PRT
   <213> Human
<400> 1
   Gly Asp Thr Val Glu Leu Thr Cys Thr Ala Ser Gln
    1               5                  10
<210> 2
   <211> 10
   <212> PRT
   <213> Human
<400> 2
   Lys Lys Ser Ile Gln Phe His Trp Lys Lys
   1 5 10
<210> 3
   <211> 9
   <212> PRT
   <213> Human
<400> 3
   Lys Glu Glu Val Gln Leu Leu Val Phe
   1 5
<210> 4
   <211> 9
   <212> PRT
   <213> Human
<400> 4
   Pro Ser Val Gln Cys Arg Ser Pro Arg
   1 5
<210> 5
   <211> 15
   <212> PRT
   <213> Human
<400> 5
   Phe Pro Leu Ala Phe Thr Val Glu Lys Leu Thr Gly Ser Gly Glu
   1 5 10 15
<210> 6
   <211> 13
   <212> PRT
   <213> Human
<400> 6
   Lys Leu Ile Ile Gln Glu Val Asn Leu Val Val Met Arg
   1 5 10